# EUROPEAN PATENT SPECIFICATION

(11) **EP 0 594 125 B1**
(45) Date of publication and mention of the grant of the patent: **09.01.2002**
(21) Application number: 93116878.5
(22) Date of filing: 19.10.1993
(51) Int. Cl.: C12N 11/00, B09B 3/00, C12S 13/00

(54) **Carrier for supporting microorganisms, soil remediating agent using carrier, and method for remediating soil**
Träger für Mikroorganismen, Bodenentgiftungsmittel die diesen Träger verwenden, und Methoden zur Entgiftung de Bodens
Support pour micro-organismes, agent de décontamination du sol utilisant le support et méthode de décontamination du sol

(30) Priority: 20.10.1992 JP 28198492; 20.10.1992 JP 28198592; 20.10.1992 JP 28198692
(43) Date of publication of application: 27.04.1994
(73) Proprietor: CANON KABUSHIKI KAISHA, Tokyo (JP)
(72) Inventor: Kozaki, Shinya, c/o CANON KABUSHIKI KAISHA, Ohta-ku, Tokyo 146 (JP); Kato, Kinya, c/o CANON KABUSHIKI KAISHA, Ohta-ku, Tokyo 146 (JP); Tanaka, Kazumi, c/o CANON KABUSHIKI KAISHA, Ohta-ku, Tokyo 146 (JP); Yano, Tetsuya, c/o CANON KABUSHIKI KAISHA, Ohta-ku, Tokyo 146 (JP); Sakuranaga, Masanori, c/o CANON KABUSHIKI KAISHA, Ohta-ku, Tokyo 146 (JP); Imamura, Takeshi, c/o CANON KABUSHIKI KAISHA, Ohta-ku, Tokyo 146 (JP)
(74) Representative: Tiedtke, Harro, Dipl.-Ing.

(56) References cited:
- EP-A- 0 228 626
- WO-A-90/01465
- WO-A-90/10079
- DE-A- 3 613 575
- FR-A- 2 501 229
- Section Ch, Week 9233, Derwent Publications Ltd., London, GB; Class D15, AN 92-273643 & JP-A-4 187 086 (B BY B KK) 3 July 1992
- CHEMICAL ABSTRACTS, vol. 108, no. 2, 11 January 1988, Columbus, Ohio, US; abstract no. 10677p, PORTIER 'CHITIN IMMOBILIZATION SYSTEMS FOR HAZARDOUS WASTE DETOXIFICATION AND BIODEGRADATION' page 293 ;column 1 ; & IMMOBILISATION IONS BIO-SORPTION, 1986 pages 229 - 243
- CHEMICAL ABSTRACTS, vol. 107, no. 19, 9 November 1987, Columbus, Ohio, US; abstract no. 170091a, PORTIER ET AL 'CONTINUOUS BIODEGRADATION AND DETOXIFICATION OF CHLORINATED PHENOLS USING IMMOBILIZED BACTERIA' page 189 ;column 1 ; & TOXIC.ASSESS., vol.1, no.4, 1986 pages 501 - 513

## Description

### BACKGROUND OF THE INVENTION

### Field of the Invention

The present invention relates to a soil remediating agent in which microorganisms having a degradation power to soil contaminants are contained in the pores of a carrier, and a method for remediating a soil which comprises administering the soil remediating agent to the soil.

### Related Background Art

In recent years, there has been the problem of environmental pollution with hydrocarbons such as aromatic hydrocarbons, paraffins and naphthenes as well as chlorinated organic compounds such as trichloroethylene and perchloroethylene. It has been strongly desired to establish a technique by which the escalation of the serious environmental pollution is prevented and already contaminated environments are purified to remediate the environments. Examples of this technique include physical/chemical treatments such as an aeration treatment, drying exposure, a vacuum pan using technique and a vacuum extraction, but they are not always advantageous from the total viewpoints of cost, operating characteristics, energy quantity to be used, treatable range and degradation properties of less degradable substances.

Thus, as a technique for solving the problem of the physical/chemical treatment, it has been investigated to utilize a biological treatment using microorganisms for the purpose of the remediation of the environments.

As the microorganisms capable of degrading less degradative substances such as aromatic hydrocarbons and chlorinated organic compounds which cause soil contamination, many kinds of microorganisms are known. However, if such bacteria are directly sprayed on the actually contaminated soil, the concentration of the bacteria gradually decreases from an initial bacteria concentration in the soil with the passage of time, so that the remediation efficiency of the contaminated soil deteriorates unsuitably. In consequence, the spray of the bacteria must often be repeated inconveniently. Therefore, it is strongly desired from a practical standpoint to retain the growth power of the degradation bacteria administered to the soil and to maintain the bacterial concentration at a high level.

As carriers for use in bioreactors which have been heretofore used in a drug manufacturing industry, a food industry, a waste water treating system and the like, there have been suggested various carriers for supporting the microorganisms and maintaining them at a high concentration. Examples of such carriers include particle carriers such as porous glasses, ceramics, metal oxides, active carbons, zeolites and anthracites as well as gel-like carriers such as agar, polyvinyl alcohol, alginic acid, polyacrylamide and carrageenan.

Japanese Kohyo Publication (Japanese National Publication of PCT Application) No. 503528/1992 discloses a method which comprises immobilizing microorganisms capable of degrading chlorophenols on a porous carrier, and then adding the carrier to contaminated soil. This kind of porous carrier can provide the microorganisms with a preferable growth circumstance and can protect them from the attack of predatory animals.

WO90/10079 discloses a process for the microbiological remediation of soil polluted by chlorophenol by means of chlorophenol-degrading microorganisms of the genus *Rhodococcus* and *Mycobacterium*. The microorganisms may be added immobilized in a solid porous organic support.

On the other hand, EP-A-0 228 626 discloses a decontamination process of soils by microorganisms wherein pollutant specific microorganisms are applied to the soil in free or fixed form. As substrates for the immobilization of the microorganisms active carbon, zeolites and silica are mentioned. A carrier material may be placed into the soil and thereafter is contacted with an aqueous suspension of a microorganism and subsequently contacted with an aqueous mineral solution.

However, the soil is usually poor in nutrition, and so it is often necessary to replenish the soil with a nutrient for the soil remediating microorganisms added to the soil. In such a case, the movement of the nutrient in the soil is very slow. Therefore, even if the nutrient for the soil remediating microorganisms is given to the soil from its surface, the effect of the nutrient cannot be rapidly shown. For this reason, if the microorganisms are directly sprayed to the soil and even if they are immobilized in the above-mentioned organic, inorganic, particulate, gel-like or porous carrier, the sufficient nutrient cannot be fed to the administered microorganisms, so that the growth power of the administered microorganisms cannot be sufficiently activated and the concentration of the bacteria cannot be sufficiently maintained in the soil.

Conversely, for example, even if conditions suitable for the administered microorganisms are given and even if the microorganisms are grown and maintained at a high concentration to successfully degrade and remove contaminants from the soil, it is not preferable that the administered microorganisms are still present in the circumstances for a long period of time even after the removal of the contaminants, because an unnecessary trouble such as a secondary contamination by the administered microorganisms themselves is apt to take place.

That is, it is ideally desirable that the bacteria introduced from the outside work only in a contaminated area and automatically disappear after the remediation of the circumstances.

### SUMMARY OF THE INVENTION

An object of the present invention is to provide a soil remediating agent in which microorganisms are held on a porous carrier for supporting microorganisms which can easily and economically feed a nutrient to the microorganisms in a soil during the remediation of the soil by the use of the microorganisms, can activate the growth power of the microorganisms in the soil, can maintain bacteria concentration at a high level, can allow the microorganisms to work only in a contaminated area, and can allow them to automatically disappear after the remediation of the soil so as to eliminate the influence of the remaining microorganisms on an ecological system.

Another object of the present invention is to provide a process for remediating a soil by the use of the soil remediating agent.

These objects can be achieved by the present invention.

According to the present invention a soil remediating agent is provided which is characterized by comprising a porous carrier which holds microorganisms having a soil contaminant degrading power and the carrier for supporting the microorganisms holds a nutrient in the pores.

The agent is made by the steps according to claim 1.

According to the present invention a method for remediating a soil is provided which comprises administering the above soil remediating agent to the soil by the steps as defined in claim 6 or claim 10.

### BRIEF DESCRIPTION OF THE DRAWINGS

Fig. 1 shows the change of a bacteria number between Example 3 and Comparative Example 1.

Fig. 2 shows the change of a bacteria number between Example 4 and Comparative Example 2.

Fig. 3A shows the change of trichloroethylene concentration between Example 5 and Comparative Example 3.

Fig. 3B shows the change of a bacteria number between Example 5 and Comparative Example 3.

Fig. 4 shows the change of trichloroethylene concentration between Example 6 and Comparative Example 4.

Fig. 5A shows the change of trichloroethylene concentration between Example 7 and Comparative Example 5.

Fig. 5B shows the change of a bacteria number between Example 7 and Comparative Example 5.

Fig. 6 shows the change of trichloroethylene concentration between Reference Example 8 and Comparative Example 6.

Fig. 7 shows the change of cresol concentration between Example 9 and Comparative Example 7.

Fig. 8 shows the change of phenol concentration between Example 10 and Comparative Example 8.

Fig. 9 shows the change of trichloroethylene concentration between Example 11 and Comparative Example 9.

Fig. 10 shows the change of trichloroethylene concentration between Example 12 and Comparative Example 10.

Fig. 11 shows an operation for administering a soil remediating agent to a soil by an aeration method

Fig. 12 shows an operation for administering a soil remediating agent to a soil by a water feed method.

### DESCRIPTION OF THE PREFERRED EMBODIMENTS

In view of the above-mentioned situations, the present inventors have investigated to develop a carrier useful to administer microorganisms for soil remediation having a contaminant compound degrading power to a soil, and as a result, they have found that the administered microorganisms in the soil can be effectively maintained and grown and can disappear after a certain period of time by the use of a carrier which has pores supporting the microorganisms therein, holds a nutrient for the microorganisms in the pores, or becomes the nutrient for the microorganisms. In consequence, the present invention has now been attained.

Now, the present invention will be described in more detail.

Reference will be made to a carrier which has pores supporting microorganisms therein and holds a nutrient for the microorganisms in the pores, and a soil remediating agent in which the microorganisms are supported in the carrier.

Examples of the fundamental constitutional material of the microorganisms-supporting carrier include polysaccharides such as cellulose, dextran and agarose, inert proteins such as gelatin and albumin, synthetic polymeric compounds such as an ion exchange resin and polyvinyl chloride, inorganic materials such as ceramics and porous glasses, and polymeric compounds for comprehensive carriers such as collagen, agar, alginic acid, carrageenan, cellulose acetate, polyacrylamide, polyvinyl alcohol, epoxy resin, photosetting resins, polyesters, polystyrene and polyurethane.

The above-mentioned fundamental constitutional material is preferably such as to be capable of forming a porous carrier which permits the microorganisms to get into its fine pores, thereby heightening the retention of the microorganisms therein and in which a pore diameter is controllable to some extent. That is, the porous structure can increase the surface area to which the useful microorganisms can adhere, and the controllability of the diameter of the pores can prevent the predation of the carrier by a protozoan or the like. In the present invention, the growth and the maintenance of the useful microorganisms can be achieved in the soil only by adding a nutrient feed into the pores of the porous carrier.

Furthermore, the formation of the carrier itself from a biodegradable material is preferable, because problems such as the secondary contamination with the remaining carrier and the influence of the administered microorganisms on the soil ecological system can be prevented. Such a biodegradable material is preferably such as to gradually degrade and disappear after the remediation treatment of the soil by the administered microorganisms. If such a carrier is used, the administered microorganisms released into the soil due to the disappear of the carrier are gradually decreased owing to competition with native predominant microorganisms in the soil, predation by protozoans and the exposure of the microorganisms to circumstances severe to grow, and finally these microorganisms disappear. As a result, the soil ecological system can be returned to a normal state.

Such a biodegradable carrier can be made of cellulose, lignin, starch, agarose, dextrin, albumin, chitin, chitosan, filter paper or wood chips. The carriers made of these materials are preferable, because they can support the microorganisms in a relatively moderate state, are easy to release the grown microorganisms, are inexpensive, and can become the nutrient for the biodegradable microorganisms themselves in a certain case.

The degradation rate of the biodegradable carrier itself can be controlled by selecting its material and properties. For example, the diameter and the morphology of the pores as well as the shape and the size of the carrier can be suitably selected in consideration of the carrier material. The factors which should be taken into consideration in selecting these requirements, i.e., the factors which have an influence on the degradation rate of the biodegradable carrier include the kind, amount and degradation activity of the microorganisms (the native microorganisms in the soil or the administered microorganisms) for degrading the carrier as well as the volume of the soil to be treated. Preferably, a period taken to degrade the contaminants and a period necessary for the degradation of the carrier are beforehand confirmed by field experiments, and the carrier is designed.

The nutrient which is supported in the above-mentioned carrier contains carbon, nitrogen and phosphorus, and one example of such a nutrient is a culture medium suitable for the growth of bacteria. The culture solution is, for example, a mixture of a meat soup, yeast extract, malt extract, bactopeptone, glucose, an inorganic salt and a mineral in a suitable ratio, and this mixing ratio can be selected in compliance with the kind of microorganisms to be supported in the carrier. In the present invention, any nutrient is utilizable in addition to the above-mentioned culture medium, so long as it contains suitable amounts of organic and inorganic nutrients. For example, fine powder obtained by drying and then grinding optional microorganisms which are collected from nature or additionally cultured may be contained as the nutrient in the carrier.

Next, a procedure according to the present invention for introducing the nutrient into the above-mentioned carrier is described.

As a technique of introducing the nutrient into a water-insoluble inorganic porous carrier such as a porous glass or ceramics, there is a method comprising
(1) a step of immersing the carrier into a solution containing the nutrient,
(2) a step of allowing the solution to thoroughly penetrate through the pores of the carrier by an ultrasonic wave treatment, and
(3) a step of heating/drying the carrier to dry and consolidate the nutrient in the pores of the carrier.

In addition, as a technique of introducing the nutrient into a gel-like comprehensive carrier of carrageenan or alginic acid, there is a method comprising
(1) a step of mixing a solution containing the nutrient with a solution containing a gelation material (e.g., carrageenan or alginic acid), and
(2) a step of gelling the mixture under the control of the shape of the carrier to obtain the desired carrier.

The above-mentioned methods for introducing the nutrient are only a part of many techniques. The nutrient can be used in a drying state or an aqueous solution, and it can be introduced into the carrier in the most suitable condition and manner in consideration of the existent state, shape and properties of the carrier.

One soil remediating agent of the present invention can be obtained by supporting the microorganisms in the carrier.

In order to support them in the carrier, a usual method can be used. For example, in the case that the carrier is in the form of a particulate solid, the microorganisms can be physically or ionically adsorbed in the carrier or covalently bonded to the carrier. In the case of the comprehensive carrier, the microorganisms may be comprehended in the carrier in the above-mentioned manner.

Next, reference will be made to other embodiments of the microorganisms-supporting carriers of the present invention, i.e., a carrier comprising the nutrient itself of the microorganisms and a soil remediating agent in which the microorganisms are supported in the carrier.

Concrete examples of the carrier comprising the nutrient of the microorganisms include a carrier comprising a natural polymer (e.g., chips of thinning lumber or scrap wood, lignocellulose-based agriculture and forestry waste such as bagasse or straws, shells of lobsters or crabs, or chitin-based marine waste such as squid backbone).

A drying/consolidating treatment according to item (3) above can usually be carried out by dehydrating the bacteria in an oven or a drier, and then drying them while they are still in the solid state. Here, a warming treatment may be employed to accelerate the drying step, but it is not always necessary. The consolidation by freeze-drying is also applicable. In order to enhance the strength of the carrier, it is desirable to dry the treated bacteria until a moisture content in the spaces among cells has not been present any more, but the moisture content of about 50% is sufficiently acceptable from a practical viewpoint.

After the drying and consolidation of the microorganisms in this way, they are suitably ground or cut into a desired particle size to prepare carrier particles. For example, the cultured microorganisms are collected by centrifugal precipitation or filtration, dried and consolidated at a predetermined temperature while they are in the solid state, and then ground or cut by means of a cutter, a mill or a blender to obtain a particulate solid. In this case, the size of the particles can be optionally selected by set conditions. Furthermore, the sample is taken out while it is in a semidrying state, and it is made particulate in this state. Afterward, in order to increase strength, it is also possible to additionally dry the obtained particles.

The thus prepared carrier has a pore diameter of about several µm to hundreds µm, depending upon the size, the shape and the like of the microorganisms utilized in the preparation of the carrier, and it also has an excellent shape as the carrier for supporting the soil remediating microorganisms. Alternatively, the optional pore diameter of about several µm to hundreds µm can also be obtained by the suitable selection of conditions for bacteria collection.

Next, the carrier which can be obtained by using the natural polymer as the raw material will be described in detail. A lignocellulose-based or chitin-based natural polymer has pores of various shapes derived from cells and is rich in carbon and nitrogen sources necessary for the existence of the microorganisms. Therefore, even if the above-mentioned natural polymer is directly used as the carrier for supporting the soil remediating microorganisms, a sufficient effect can be obtained.

Furthermore, these carriers have the pores suitable to support the microorganisms for the soil remediation, and therefore they have a function enough to grow and maintain the microorganisms. The carrier comprising the nutrient means that it is biodegradable. This kind of carrier has sufficient strength, even if brought into contact with water and swelled. In addition, the carrier is not divided into fine parts, even if sprayed in the contaminated soil, and therefore the thus prepared carrier can be considered to be practicable.

The soil remediating agent of the present invention can be obtained by introducing the microorganisms into the above-mentioned carrier.

In order to introduce the microorganisms for soil remediation into the carrier, a usual technique can be utilized, and there can be used a method which comprises mixing the carrier and a culture medium, and then culturing the microorganisms, or a method which comprises confining the microorganisms to the pores under reduced pressure.

No particular restriction is put on the microorganisms which are supported in the carrier of the present invention, but examples of the usable microorganisms include bacteria belonging to Pseudomonas genus for degrading aromatic hydrocarbon compounds, an organic solvent such as toluene and chlorinated organic compounds, and microorganisms belonging to the genera of Methylosinus, Methylomonas, Methylobacterium, Alcaligenes, Mycobacterium, Nitrosomonas, Xanthomonas, Spirillum, Vibrio, Bacterium, Achromobacter, Acinetobacter, Flavobacterium, Chromobacterium, Desulfovibrio, Desulfotomaculum, Micrococcus, Sarcina, Bacillus, Streptomyces, Nocardia, Corynebacterium, Pseudobacterium, Arthrobacter, Brevibacterium, Saccharomyces and Lactobacillus which are known to have a degrading ability to various kinds of harmful substances.

As the microorganisms to be administered, there can be utilized microorganisms already isolated, microorganisms newly screened from a soil or the like in compliance with a purpose, or a mixture of a plurality of strains. The screened microorganisms may be unidentified.

The purification treatment of the soil can be carried out by administering the carrier supporting the microorganisms or the soil remediating agent of the present invention to the soil. The administration of the carrier or the soil remediating agent to the soil can be done by a usual manner such as spraying or mixing with the soil. In order to administer it to a relatively deep portion of the soil, a method can be utilized which comprises digging a hole, and then administering/dispersing the soil remediating agent. One embodiment of such a method will be described in reference to attached drawings.

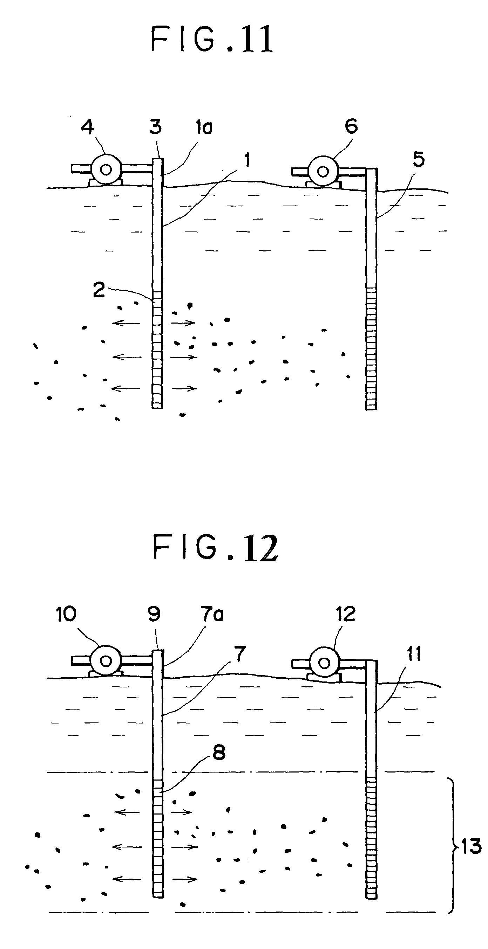

Fig. 11 shows the administration method utilizing an aeration hole. In the practice of the administration method utilizing the aeration hole, an aeration hole 1 is first formed in a soil by a means such as digging, and an aeration pipe 1a is inserted into the aeration hole 1. Afterward, pressure is applied to the interior of the aeration pipe 1a by an air blowing means such as a compressor to generate positive pressure, and the soil remediating agent is fed through a feed inlet 3 and is then released into the soil through many pits 2 for releasing the soil remediating agent of the aeration pipe 1a. If necessary, a suction hole 5 is provided at a suitable position, and the interior of the suction hole is made negative pressure by a suction means 6 such as a suction pump, whereby the dispersion effect of the soil remediating agent by the aeration in the aeration hole 1 can be accelerated. In general, oxygen partial pressure in a deep portion of the soil is low, which is often unsuitable for the treatment by the use of aerobic bacteria. According to this method using the aeration hole, however, oxygen can be fed to the deep portion of the soil, so that the active degradating function of the aerobic bacteria can be obtained. In this case, the size (internal diameter), depth and shape of the aeration hole and the suction hole, the feed of air to the aeration hole, and the amount of air to be sucked through the suction hole can be suitably selected in compliance with the depth of the soil layer to be treated, soil quality, kinds of microorganisms and carrier, and the like.

Furthermore, it is also possible to feed the soil remediating agent to the soil by the utilization of water supply.

For example, as shown in Fig. 12, a water supply hole 7 is provided, and a water supply pipe 7a is then inserted into the hole 7. Afterward, water is fed to the water supply pipe 7a from a water supply means 10 such as a water supply pump to apply water pressure, and the soil remediating agent is fed through a feed inlet 9, forwarded together with the fed water, and then dispersed into a deep portion of the soil through pits 8. If the pits 8 are disposed in an underground water vein 13, the soil remediating agent can be dispersed into the soil by the utilization of an underground water flow. When water containing a large amount of dissolved oxygen is used as water to be supplied by the water supply means 10, oxygen can also be fed to the soil, thereby obtaining an active degradating function by the aerobic bacteria. Furthermore, if necessary, a water suction hole 11 is provided, and water is sucked up by a water suction means 12 such as a water suction pump to accelerate the effect of the above-mentioned water supply. In this method utilizing underground water, the size (internal diameter), depth and shape of the water supply hole and the water suction hole, the feed of water to the water supply hole, and the amount of water to be sucked through the water supply hole can be suitably selected in compliance with the depth of the soil layer to be treated, soil quality, kinds of microorganisms and carrier, and the like.

Now, the present invention will be described in more detail in reference to examples, but the scope of the present invention should not be limited to these examples at all.

### Example 1

### (Preparation of a microorganisms-supporting carrier from a porous glass)

A porous glass MPG (pore diameter 20 µm, particle diameter 2-3 mm, and 0.35 cc/g) made by Asahi Glass Co., Ltd. was used as a carrier. Next, 30 ml of a YPD culture medium (containing 10 g of yeast extract, 20 g of peptone, 20 g of glucose and water per liter) which was a culture medium for yeast was added to 20 g of this MPG, and then treated for 1 minute by an ultrasonic washer. Afterward, the excessive culture medium was removed, and the carrier was then dried at 70°C for 2 hours by the use of a drier to prepare the microorganisms-supporting carrier holding the nutrient.

### Reference Example 2

The Reference Examples are useful for understanding the present invention but are outside of its scope.

### (Preparation of a soil remediating agent from polyvinyl alcohol)

5 g of polyvinyl alcohol (average polymerization degree 2,000) was dissolved in 50 ml of a YPD culture medium just taken out from an autoclave, and after cooling, 5 g of sodium hydrogencarbonate and 5 g of yeast (S. cerevisiae AB 1380 strain, made by Toyobo Co., Ltd.) wet bacteria were mixed therewith to obtain a mixture of the polyvinyl alcohol, the yeast and the nutrient for the yeast. Afterward, this mixture was added dropwise to 60% ammonium sulfate (pH 4), and after contact for about 30 minutes, the mixture was washed with water to prepare a soil remediating agent supporting the nutrient and the microorganisms (yeast).

### Example 3

### [Survival properties (1) of Saccharomyces cerevisiae in a soil]

S. cerevisiae YPH 499 strain was inoculated into 10 ml of a YPD culture medium, and the bacteria were then cultured at 30°C until a logarithmic growth phase was reached. Next, 1 g of a microorganisms supporting carrier prepared in Example 1 was added to this culture medium, and then placed in a pressure bottle. Afterward, the pressure in the bottle was reduced to introduce the bacterial into the carrier, thereby preparing a soil remediating agent.

The number of the bacteria introduced into the carrier was determined from an absorbance-bacteria number linearity. That is, the number of the bacteria before their introduction into the carrier and the number of the bacteria after their introduction into the carrier were determined from the absorbance, and a difference between the determined numbers was regarded as the number of the bacteria introduced into the carrier. The absorbance-bacteria number linearity was obtained by counting the number of the bacteria in a bacteria suspension having a certain absorbance by the use of a hemocytometer.

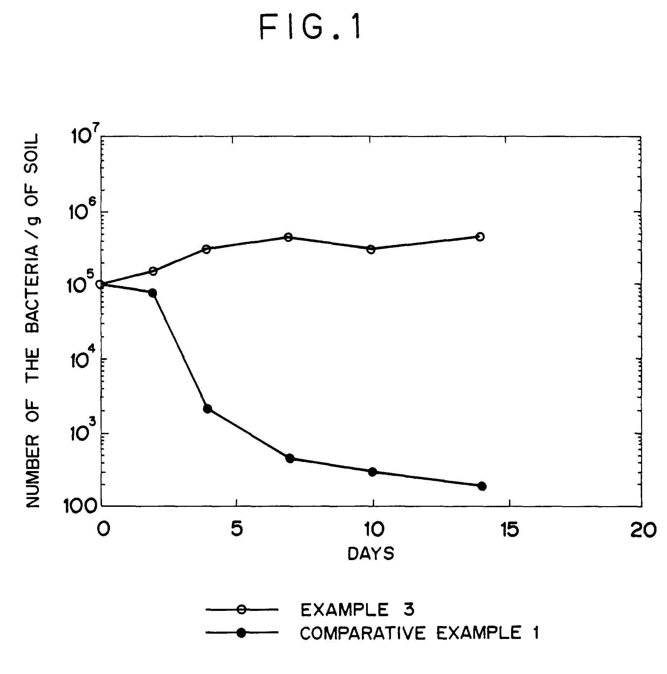

The thus prepared soil remediating agent was added to 100 g of a sterilized brown forest soil, and the bacteria were cultured at 30°C. Afterward, the soil was sampled every 10 g with day. The sample was dispersed into 50 ml of a phosphoric acid buffer solution by the use of a homogenizer, and the number of the bacteria was determined in accordance with a dilution plate technique of the YPD culture medium. In this case, 5 experimental systems having the same constitution were prepared and experimented with 5 runs, and the number of the bacteria per gram of the soil was determined on the average. The results are shown in Fig. 1.

### Comparative Example 1

The same experimental system as in Example 3 was used except that MPG to which any nutrient was not added was used as a carrier. Furthermore, the number of yeast bacteria to be introduced into a soil was set so as to be about the same as in Example 3. The soil was sampled with day as in Example 3 to count the number of the bacteria. The experiment was carried out with 5 runs, and the number of the bacteria per gram of the soil was determined on the average. The results are shown in Fig. 1.

### Reference Example 4

### [Survival properties (2) of Saccharomyces cerevisiae in a soil]

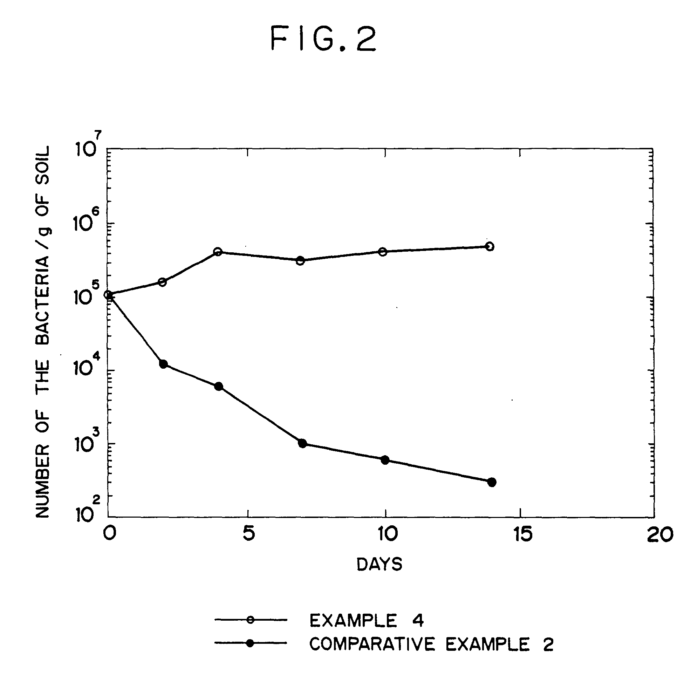

The same experimental system as in Example 3 was used except that the soil remediating agent of Example 3 was replaced with a soil remediating agent prepared in the manner of Reference Example 2. Furthermore, the number of S. cerevisiae to be introduced into the soil was set so as to be about the same as in Example 3. The soil was sampled with day as in Example 3 to count the number of the bacteria. The experiment was carried out with 5 runs, and the number of the bacteria per gram of the soil was determined on the average. The results are shown in Fig. 2.

### Comparative Example 2

The same experimental system as in Example 4 was used except that a soil remediating agent was prepared without using a YPD culture medium, that is, a soil remediating agent containing no culture medium was used. Furthermore, the number of S. cerevisiae to be introduced into the soil was set so as to be about the same as in Example 3. The soil was sampled with day as in Example 3 to count the number of the bacteria. The experiment was carried out with 5 runs, and the number of the bacteria per gram of the soil was determined on the average. The results are shown in Fig. 2.

### Example 5

### [Degradation properties of trichloroethylene (TCE) by Pseudomonas cepacia and Survival properties (1) of Pseudomonas cepacia]

### (Preparation of a microorganisms supporting carrier from cellulose)

500 ml of an M9 culture medium (containing 6.2 g of NaHPO₄, 3.0 g of KH₂PO₄, 0.5 g of NaCl, 1.0 g of NH₄Cl and distilled water per liter and having a pH of 7.0) containing 0.5% of yeast extract was added to 20 g (dry weight) of a cellulose fine powder carrier (microcarrier made by Asahi Chemical Industry Co., Ltd.) as a porous carrier, and air in the carrier was then removed by reducing pressure. Afterward, the mixture was treated for 1 minute by an ultrasonic washer. Next, the excessive culture medium was removed, and the carrier was then dried at 70°C for 2 hours by the use of a drier to prepare the microorganisms supporting carrier holding the nutrient.

### (Degradation of trichloroethylene)

Pseudomonas cepacia KK01 (International Deposition No. BP-4235) which were bacteria for degrading trichloroethylene and phenol were inoculated into 1,000 ml of an M9 culture medium containing 0.05% of yeast extract, and the bacteria were then cultured at 30°C. After O.D. (530 nm) of the culture medium had reached about 0.7, 20 g (dry weight) of the above-mentioned microorganisms supporting carrier was added to this solution, and then placed in a pressure bottle. Afterward, the pressure in the bottle was reduced to introduce the bacterial into the carrier.

This carrier was spread on a filter paper (Wattman No. 44), and the excessive culture medium was then removed from the carrier by suction. Next, 100 ml of distilled water was poured onto the carrier on the filter paper under suction three times to wash out a small number of the bacteria which were not adsorbed in the carrier.

The number of the bacteria which were adsorbed in the carrier was determined as follows. In the first place, 10 g of the carrier having the adsorbed bacteria was placed in a blender cup, and 40 ml of sterilized water was added thereto, followed by a blender treatment at 10,000 rpm for 1 minute. The number of the bacteria in the thus obtained bacteria suspension was determined in accordance with a dilution plate technique. As a culture medium, a phenol single carbon source agar culture medium was used, and a colony state was distinguished and plate count was then carried out.

Next, 1 mg of trichloroethyelene and 20 mg of phenol which was an inducer material for TCE degradation were added to 50 g of an unsterilized brown forest soil having a moisture content of 80%, and 5 g (wet weight) of a soil remediating agent prepared in the above-mentioned manner was added to and mixed with the forest earth in a 100 ml vial bottle and this vial bottle was then sealed with a butyl rubber stopper. The same three samples were prepared, and then allowed to stand in an incubator at 20°C. Moreover, 50 ppm of phenol was added thereto through the rubber stopper after measurement every 7 days by a syringe.

Zero day, 7 days, 14 days, 21 days and 28 days after the start of the cultivation, the concentration of trichloroethylene in a gaseous phase in each vial bottle was measured by a gas chromatography-headspace method. Each experiment was carried out with 3 runs. The average values of the trichloroethylene concentration were calculated, and relative values of the remaining trichloroethylene were then determined by regarding an initial concentration as 100.

Zero day, 7 days, 14 days, 21 days, 28 days, 56 days, 84 days and 112 days after the soil which was mixed with the contaminants and the soil remediating agent containing the bacteria was cultured, the number of the remaining bacteria was similarly measured. This measurement was carried out by suspending the soil by a blender, and then counting the number of the bacteria in the suspension in accordance with the above-mentioned dilution plate technique. Incidentally, the cellulose carrier began to suffer a biodegradation function from the 56th day or so.

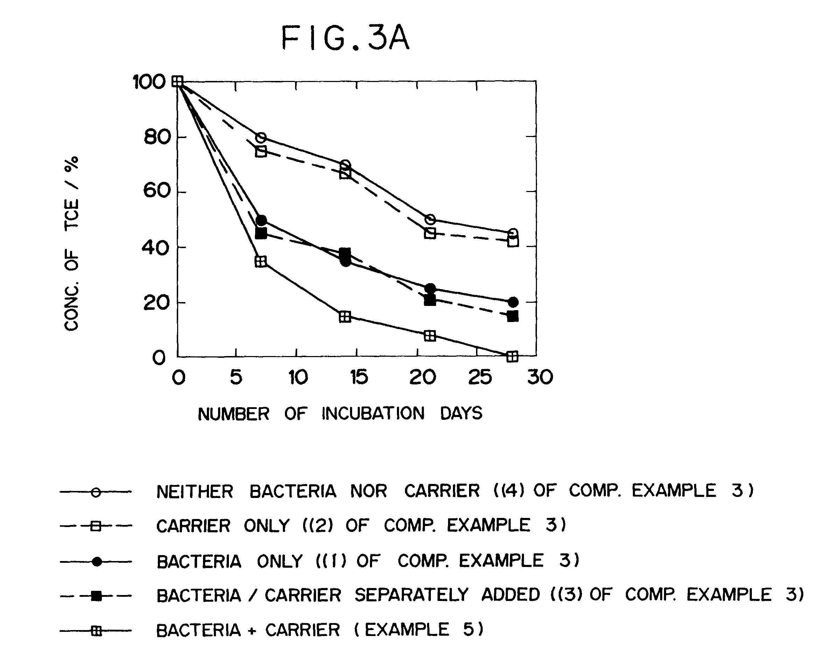

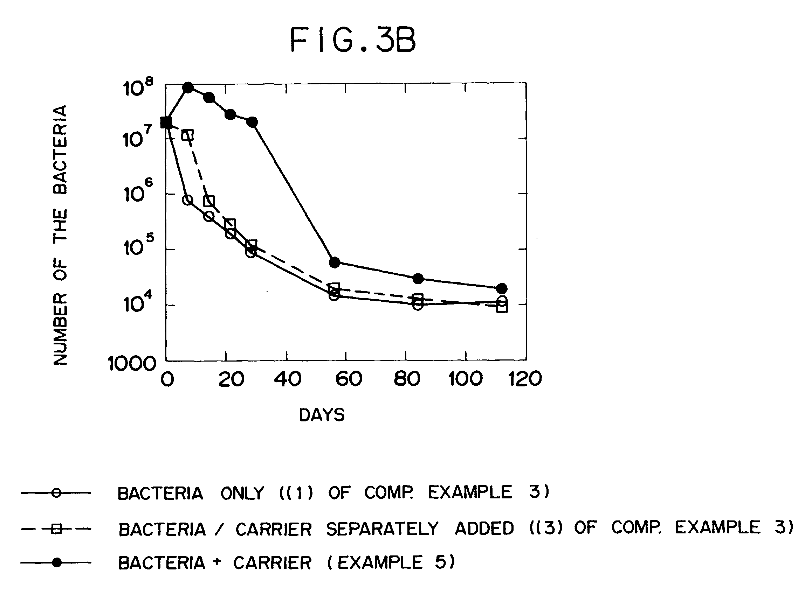

These results are shown in Figs. 3A and 3B.

### Comparative Example 3

The following systems were first prepared: (1) a system formed by mixing a soil with 5 ml of a bacteria suspension prepared by the utilization of absorbance in the same manner as in Example 5 so that the number of the bacteria in the system might be about the same as the number of the bacteria adsorbed in a carrier of Example 5, (2) a system formed by mixing a soil with 5 g of a carrier having no adsorbed bacteria obtained by wetting a microorganisms supporting carrier holding a nutrient with distilled water, and then sucking the carrier through a filter paper in the same manner as in Example 5 to remove excessive distilled water, (3) a system formed by mixing a soil separately with 5 ml of a bacteria suspension prepared by the utilization of absorbance so that the number of the bacteria in the system might be about the same as the number of the bacteria adsorbed in a carrier of Example 5 and 5 g of a carrier having no adsorbed bacteria, and (4) a system formed by mixing 5 ml of distilled water with a soil. Next, each system was sealed and cultured as in Example 5, and the concentration of trichloroethylene in each vial bottle was measured with day by a gas chromatography-headspace method. Each experiment was carried out with 3 runs. The average values of the trichloroethylene concentration were calculated, and relative values of the remaining trichloroethylene were then determined by regarding an initial concentration as 100.

Zero day, 7 days, 14 days, 21 days, 28 days, 56 days, 84 days and 112 days after the bacteria were cultured, the number of the remaining bacteria was measured in the same manner as in Example 1. Incidentally, the cellulose carrier began to suffer a biodegradation function from the 56th day or so.

These results are shown in Figs. 3A and 3B.

### Example 6

### (Degradation properties of trichloroethylene by Pseudomonas putida)

### (Preparation of a microorganisms supporting carrier from a zeolite)

50 ml of an M9 culture medium containing 0.5% of yeast extract was added to 30 g of a zeolite (particle diameter 200-500 µm) as an inorganic porous carrier, and then treated for 1 minute by an ultrasonic washer. Afterward, the excessive culture medium was removed, and the carrier was then dried at 70°C for 2 hours by the use of a drier to prepare the microorganisms supporting carrier holding the nutrient.

### (Degradation of trichloroethylene)

Pseudomonas putida BH (Environmental Engineering, the department of engineering, Osaka University) which were bacteria for degrading trichloroethylene and phenol were inoculated into 100 ml of an M9 culture medium containing 0.05% of yeast extract, and the bacteria were then cultured at 30°C. After O.D. (530 nm) of the culture medium had reached about 0.7, 20 g of the above-mentioned microorganisms supporting carrier was added to this solution, and then placed in a pressure bottle. Afterward, the pressure in the bottle was reduced to introduce the bacterial into the carrier.

This carrier was spread on a filter paper (Wattman No. 44), and the excessive culture medium was then removed from the carrier by suction. Next, 100 ml of distilled water was poured onto the carrier on the filter paper under suction three times to wash out a small number of the bacteria which were not adsorbed in the carrier.

The number of the bacteria which were adsorbed in the carrier was determined as follows. In the first place, 10 g of the carrier having the adsorbed bacteria was placed in a blender cup, and 40 ml of sterilized water was added thereto, followed by a blender treatment at 10,000 rpm for 1 minute. The number of the bacteria in the thus obtained bacteria suspension was determined in accordance with a dilution plate technique. As a culture medium, a phenol single carbon source agar culture medium was used, and a colony state was distinguished and plate count was then carried out.

Next, 1 mg of trichloroethyelene and 20 mg of phenol which was an inducer material for TCE degradation were added to 50 g of an unsterilized brown forest soil having a moisture content of 80%, and 5 g (wet weight) of a soil remediating agent prepared in the above-mentioned manner was mixed with the forest earth in a 100 ml vial bottle and this vial bottle was then sealed with a butyl rubber stopper. The same three samples were prepared, and allowed to stand in an incubator at 20°C. Moreover, 50 ppm of phenol was added thereto through the rubber stopper after measurement every 7 days by a syringe.

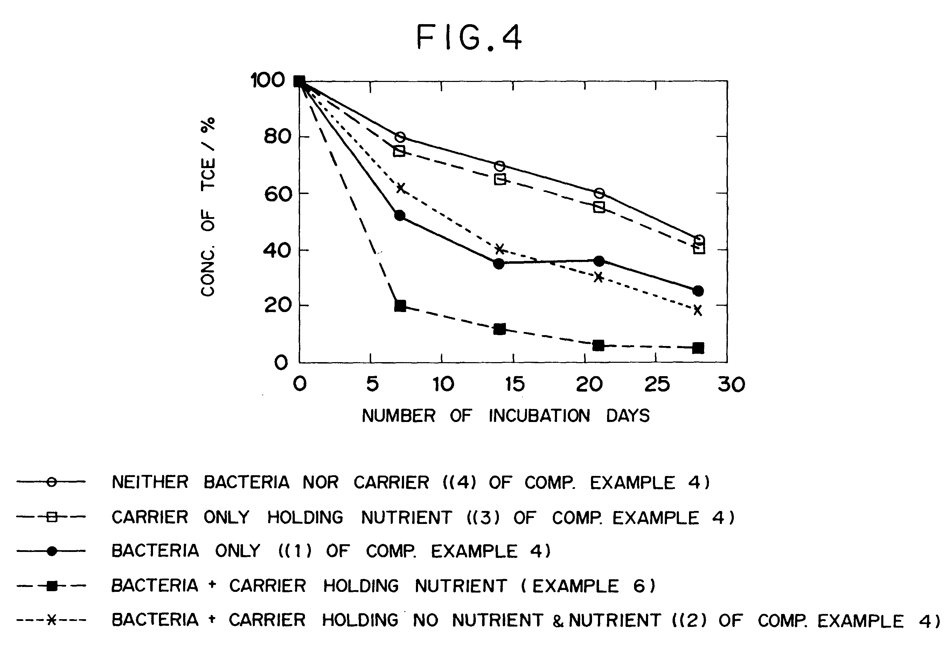

Zero day, 7 days, 14 days, 21 days and 28 days after the start of the cultivation, the concentration of trichloroethylene in a gaseous phase in each vial bottle was measured by a gas chromatography-headspace method. Each experiment was carried out with 3 runs. The average values of the trichloroethylene concentration were calculated, and relative values of the remaining trichloroethylene were then determined by regarding an initial concentration as 100. These results are shown in Fig. 4.

### Comparative Example 4

The following systems were first prepared: (1) a system formed by mixing a soil with 5 ml of a bacteria suspension prepared by the utilization of absorbance so that the number of the bacteria in the system might be about the same as the number of the bacteria adsorbed in a carrier of Example 6, (2) a system formed by mixing a soil separately with 5 g of a carrier holding no nutrient (M9 culture medium) and having bacteria adsorbed in the same manner as in Example 6 and 5 ml of an M9 culture medium, (3) a system formed by mixing a soil with 5 g of a carrier having no adsorbed bacteria, and (4) a system formed by mixing a soil with 5 ml of distilled water. Next, each system was sealed and cultured as in Example 2, and the concentration of trichloroethylene in each vial bottle was measured with day by a gas chromatography-headspace method. Each experiment was carried out with 3 runs. The average values of the trichloroethylene concentration were calculated, and relative values of the remaining trichloroethylene were then determined by regarding an initial concentration as 100. These results are shown in Fig. 4.

### Example 7

### [Degradation properties of trichloroethylene by Pseudomonas cepacia and survival properties of Pseudomonas cepacia (2)]

### (Preparation of a microorganisms supporting carrier from a porous glass)

A porous glass (MPG, made by Asahi Glass Co., Ltd., pore diameter 20 µm, particle diameter 2-3 mm, and 0.35 cc/g) was used as an inorganic porous carrier. 100 ml of an M9 culture medium containing 0.5% of yeast extract was added to 60 g of MPG, and then treated for 1 minute by an ultrasonic washer. Afterward, the excessive culture medium was removed, and the carrier was then dried at 70°C for 2 hours by the use of a drier to prepare the microorganisms supporting carrier holding the nutrient.

### (Degradation of trichloroethylene)

Pseudomonas cepacia KK01 (International Deposition No. BP-4235) were inoculated into 200 ml of an M9 culture medium containing 0.05% of yeast extract, and the bacteria were then cultured at 30°C. After O.D. (530 nm) of the culture medium had reached about 0.7, 60 g of the above-mentioned microorganisms supporting porous carrier was added to this solution, and then placed in a pressure bottle. Afterward, the pressure in the bottle was reduced to introduce the bacterial into the fine pores of the carrier.

This carrier was spread on a filter paper (Wattman No. 44), and the excessive culture medium was then removed from the carrier by suction. Next, 100 ml of distilled water was poured onto the carrier on the filter paper under suction three times to wash out a small number of the bacteria which were not adsorbed in the carrier.

The number of the bacteria which were adsorbed in the carrier was determined as follows. In the first place, 10 g of the carrier having the adsorbed bacteria was placed in a blender cup, and 40 ml of sterilized water was added thereto, followed by a blender treatment at 10,000 rpm for 1 minute. The number of the bacteria in the thus obtained bacteria suspension was determined in accordance with a dilution plate technique. As a culture medium, a phenol single carbon source agar culture medium was used, and a colony state was distinguished and plate count was then carried out.

Next, 1 mg of trichloroethyelene and 20 mg of phenol which was an inducer material for TCE degradation were added to 50 g of an unsterilized brown forest earth having a moisture content of 80%, and 5 g (wet weight) of a soil remediating agent prepared in the above-mentioned manner was mixed with the forest earth in a 100 ml vial bottle and this vial bottle was then sealed with a butyl rubber stopper. The same three samples were prepared, and allowed to stand in an incubator at 20°C. Moreover, 50 ppm of phenol was added thereto through the rubber stopper after measurement every 7 days by a syringe.

Zero day, 7 days, 14 days, 21 days and 28 days after the start of the cultivation, the concentration of trichloroethylene in a gaseous phase in each vial bottle was measured to obtain average values, and a relative value of the remaining trichloroethylene was determined by regarding an initial concentration as 100.

Zero day, 7 days, 14 days, 21 days and 28 days after the soil which was mixed with the contaminants and the soil remediating agent containing the bacteria was cultured, the number of the remaining bacteria was similarly measured. This measurement was carried out by suspending the soil by a blender, and then counting the number of the bacteria in the suspension in accordance with the above-mentioned dilution plate technique.

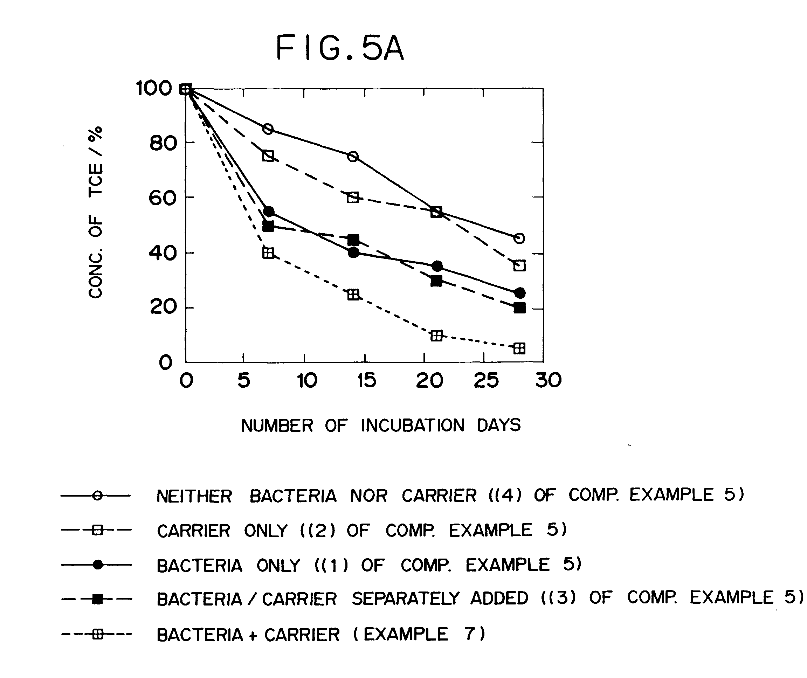

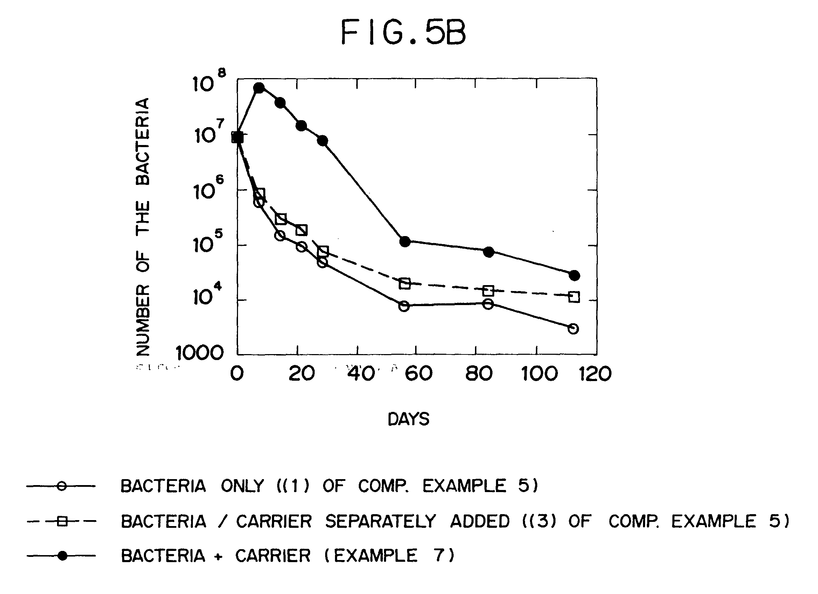

These results are shown in Figs. 5A and 5B.

### Comparative Example 5

The following systems were first prepared: (1) a system formed, in the same manner as in Example 7, by mixing a soil with 5 ml of a bacteria suspension prepared by the utilization of absorbance so that the number of the bacteria in the system might be about the same as the number of the bacteria adsorbed on a carrier of Example 7, (2) a system formed by mixing a soil with 5 g of a carrier having no adsorbed bacteria obtained by wetting a microorganisms supporting carrier holding a nutrient with distilled water, and then sucking the carrier through a filter paper in the same manner as in Example 7 to remove excessive distilled water, (3) a system formed by mixing a soil separately with 5 ml of a bacteria suspension prepared by the utilization of absorbance so that the number of the bacteria in the system might be about the same as the number of the bacteria adsorbed on a carrier of Example 7 and 5 g of a carrier having no adsorbed bacteria, and (4) a system formed by mixing a soil with 5 ml of distilled water. Next, each system was sealed and cultured, and the concentration of trichloroethylene in each vial bottle was measured with day by a gas chromatography-headspace method, as in Example 7. Each experiment was carried out with 3 runs. The average values of the trichloroethylene concentration were calculated, and relative values of the remaining trichloroethylene were then determined by regarding an initial concentration as 100.

Zero day, 7 days, 14 days, 21 days and 28 days after the bacteria were cultured, the number of the remaining bacteria in the soil was measured in the same manner as in Example 1.

These results are shown in Figs. 5A and 5B.

### Reference Example 8

### [Degradation properties of trichloroethylene by Pseudomonas cepacia (3)]

20 g of polyvinyl alcohol (average polymerization degree 2,000) was dissolved in 200 ml of an M9 culture medium containing 0.05% of yeast extract just taken out from an autoclave, and after cooling, 20 g of sodium hydrogen carbonate and 5 g of Pseudomonas cepacia wet bacteria were mixed therewith to obtain a mixture of the polyvinyl alcohol, Pseudomonas cepacia and the nutrient. Afterward, this mixture was added dropwise to 60% ammonium sulfate (pH 4), and after contact for about 30 minutes, the mixture was washed with water to prepare a soil remediating agent.

Next, 1 mg of trichloroethyelene and 20 mg of phenol which was an inducer material for TCE degradation were added to 50 g of an unsterilized brown forest earth having a moisture content of 80%, and 5 g of a soil remediating agent prepared in the above-mentioned manner was mixed with the above-mentioned forest earth in a 100 ml vial bottle and this vial bottle was then sealed with a butyl rubber stopper. The same three samples were prepared, and allowed to stand in an incubator at 20°C. Moreover, 50 ppm of phenol was added thereto through the rubber stopper after measurement every 7 days by a syringe.

Zero day, 7 days, 14 days, 21 days and 28 days after the start of the cultivation, the concentration of trichloroethylene in a gaseous phase in each vial bottle was measured by a gas chromatography-headspace method. Each experiment was carried out with 3 runs. The average values of the trichloroethylene concentration were calculated, and relative values of the remaining trichloroethylene were then determined by regarding an initial concentration as 100.

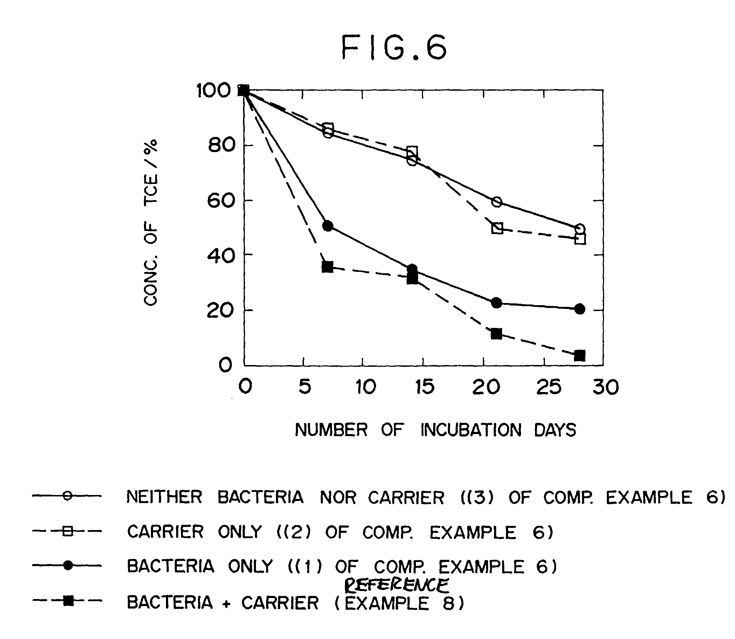

The results are shown in Fig. 6.

### Comparative Example 6

A soil was mixed with (1) 5 ml of a bacteria suspension obtained by suspending, in 200 ml of an M9 culture medium, 5 g of P. cepacia wet bacteria which was the same amount as in Example 8, (2) 5 g of a polyvinyl alcohol formed without mixing any bacteria and (3) 5 ml of distilled water, as in Example 8. After sealing, the bacteria were similarly cultured, and the concentration of trichloroethylene in a gaseous phase in each vial bottle was measured with day by a gas chromatography-headspace method. Each experiment was carried out with 3 runs. The average values of the trichloroethylene concentration were calculated, and relative values of the remaining trichloroethylene were then determined by regarding an initial concentration as 100. The results are shown in Fig. 6.

### Example 9

### (Degradation properties of cresol by Pseudomonas cepacia)

### (Preparation of a microorganisms supporting carrier from wood powder)

Wood powder was used as a porous carrier. 50 ml of an M9 culture medium containing 0.5% of yeast extract was added to 30 g of this carrier, and then treated for 1 minute by an ultrasonic washer. Afterward, the excessive culture medium was removed, and the carrier was then dried at 70°C for 2 hours by the use of a drier to prepare the microorganisms supporting carrier holding the nutrient.

### (Degradation of cresol)

Pseudomonas cepacia KK01 (International Deposition No. BP-4235) which were bacterial capable of degrading cresol was inoculated into 300 ml of an M9 culture medium containing 0.05% of yeast extract, and the bacteria were then cultured at 30°C. After O.D. (530 nm) of the culture medium had reached about 0.7, 60 g of the above-mentioned microorganisms supporting carrier was added to this solution, and then placed in a pressure bottle. Afterward, the pressure in the bottle was reduced to introduce the bacterial into the fine pores of the carrier.

This carrier was spread on a filter paper (Wattman No. 44), and the excessive culture medium was then removed from the carrier by suction. Next, 100 ml of distilled water was poured onto the carrier on the filter paper under suction three times to wash out a small number of the bacteria which were not adsorbed in the carrier.

The number of the bacteria which were adsorbed in the carrier was determined as follows. In the first place, 10 g of the carrier having the adsorbed bacteria was placed in a blender cup, and 40 ml of sterilized water was added thereto, followed by a blender treatment at 10,000 rpm for 1 minute. The number of the bacteria in the thus obtained bacteria suspension was determined in accordance with a dilution plate technique. As a culture medium, a phenol single carbon source agar culture medium was used, and a colony state was distinguished and plate count was then carried out.

Next, 5 g of a soil remediating agent prepared in the above-mentioned manner was mixed with 50 g of an unsterilized brown forest earth containing 20 mg of cresol and having a moisture content of 80% in a 100 ml vial bottle, and this vial bottle was then sealed with a butyl rubber stopper. The same 15 samples were prepared, and allowed to stand in an incubator at 20°C. Moreover, 50 ppm of phenol was added thereto through the rubber stopper after measurement every 7 days by a syringe. Zero day, 7 days, 14 days, 21 days and 28 days after the start of the cultivation, the concentration of cresol in the soil was determined by measuring a soil extract by HPLC. Each experiment was carried out with 3 runs. The average values of the trichloroethylene concentration were calculated, and relative values of the remaining trichloroethylene were then determined by regarding an initial concentration as 100.

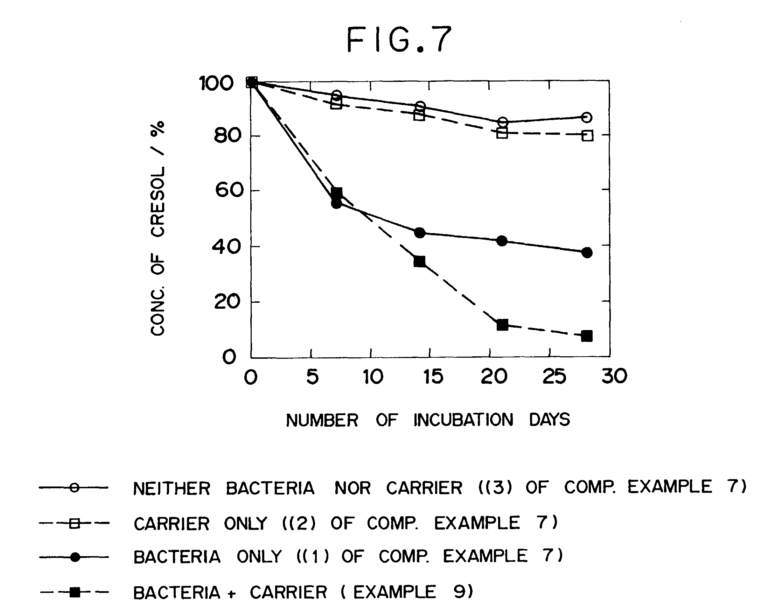

The results are shown in Fig. 7.

### Comparative Example 7

A soil was mixed with (1) 5 ml of a bacteria suspension prepared by the utilization of absorbance so that the number of the bacteria in the system might be about the same as the number of the bacteria adsorbed in a carrier of Example 9, (2) 5 g of a carrier having no adsorbed bacteria, and (3) 5 ml of distilled water, as in Example 9. After sealing, the bacteria were similarly cultured, and the concentration of trichloroethylene in a gaseous phase in each vial bottle was measured with day by a gas chromatography-headspace method. Each experiment was carried out with 3 runs. The average values of the trichloroethylene concentration were calculated, and relative values of the remaining trichloroethylene were then determined by regarding an initial concentration as 100. The results are shown in Fig. 7.

### Example 10

### [Purification of a contaminated soil (1)]

A brown forest soil was mixed with phenol in such a ratio as to disperse 80 ml of a 1,500 ppm aqueous phenol solution to 500 g of the soil, thereby preparing a phenol-contaminated soil.

An underground tank having a length of 2 m, a width of 2 m and a depth of 3 m was filled with this contaminated soil until a layer thickness of 1 m had been reached, and a brown forest soil was then superposed upon this contaminated soil layer to form a soil layer (two-layer structure) having a depth of 2.5 m. Next, aeration holes having a depth of 2.5 m and a diameter of 5 cm were provided at four corners in this test soil tank, and pipes were inserted into these holes. These pipes were made of polyvinyl chloride, had an external diameter of 40 mm, and were provided with many aeration pits having a diameter of 1 mm within the range of 1 m from its tip. The outer periphery of each pipe inlet was sealed with clay to form aeration lines. Furthermore, a pipe having a diameter of 60 mm and many aeration pits within the range of 1 m from its tip was disposed at the central position of the tank in like manner to provide a suction line. The above-mentioned aeration lines were connected to a compressor, and aeration was carried out at about 0.2 m²/min. In addition, the suction line is connected to a suction pump, and suction was done at about 0.5 m²/min.

On the other hand, 100 g of a microorganisms supporting carrier holding a cellulose nutrient which was prepared in the same manner as in Example 5 was dispersed in 5 liters of a culture medium (which was prepared by adding 0.05% of yeast extract to an M9 culture medium), and a Pseudomonas cepacia KK01 strain was inoculated into the culture medium, followed by stationary culture for 48 hours.

This microorganism culture medium containing the soil remediating agent was inoculated into the prepared soil layer in the soil tank, while aeration and suction were continuously carried out. This inoculation was given every day over one week.

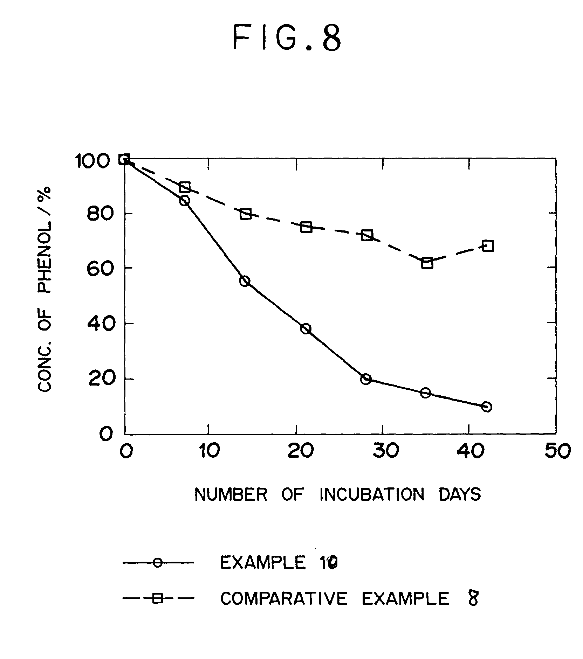

After the inoculation, the soil (at 5 positions in all) was sampled in the vicinity of each aeration line every one week, and a phenol concentration in the soil was quantitatively analyzed by HPLC and a remaining phenol amount was determined from average values at the 5 positions of the soil. The obtained results are shown in Fig. 8.

### Comparative Example 8

The same procedure as in Example 10 was repeated except that a microorganisms supporting carrier holding a cellulose nutrient was not used, to determine the change of the remaining phenol amount. The obtained results are shown in Fig. 8.

### Example 11

### [Purification of a contaminated soil (2)]

A brown forest soil was mixed with trichloroethylene in such a ratio as to disperse 8 ml of a 60 ppm aqueous trichloroethylene solution to 500 g of the soil, thereby preparing a trichloroethylene-contaminated soil. An underground tank having a length of 2 m, a width of 2 m and a depth of 3 m was filled with this contaminated soil until a layer thickness of 1 m had been reached, and a brown forest soil was superposed upon this contaminated soil layer to form a soil layer (two-layer structure) having a depth of 2.5 m.

Next, water supply holes having a depth of 2.5 m and a diameter of 5 cm were provided at four corners in this test soil tank, and pipes were inserted into these water supply holes. These pipes were made of polyvinyl chloride, had an external diameter of 50 mm, and were provided with many water supply pits having a diameter of 5 mm within the range of 1 m from its tip. The outer periphery of each pipe inlet was sealed with clay to form water supply lines. Furthermore, a pipe having a diameter of 50 mm and many aeration pits within the range of 1 m from its tip was disposed at the central position of the tank in like manner to provide a water suction line. The above-mentioned water supply lines were connected to a water supply pump, and water was supplied at about 0.5 liter/min. In addition, the water suction line is connected to a water suction pump, and water was sucked at about 5 liters/min.

On the other hand, 100 g of a microorganisms support carrier holding a wood powder nutrient which was prepared in the same manner as in Example 9 was dispersed in 5 liters of a culture medium (which was prepared by adding 0.05% of yeast extract to an M9 culture medium), and a Pseudomonas cepacia KK01 strain was inoculated into the culture medium, followed by stationary culture for 48 hours. This microorganism culture medium containing the soil remediating agent was inoculated into the prepared soil layer in the soil tank through the water supply line. This inoculation was given every day over one week.

After the inoculation, the soil (at 5 positions in all) was sampled in the vicinity of the water supply lines every one week, and a trichloroethylene concentration in the soil was quantitatively analyzed by a solvent extraction method and a remaining phenol amount was determined from average values at the 5 positions of the soil. The obtained results are shown in Fig. 19.

### Comparative Example 9

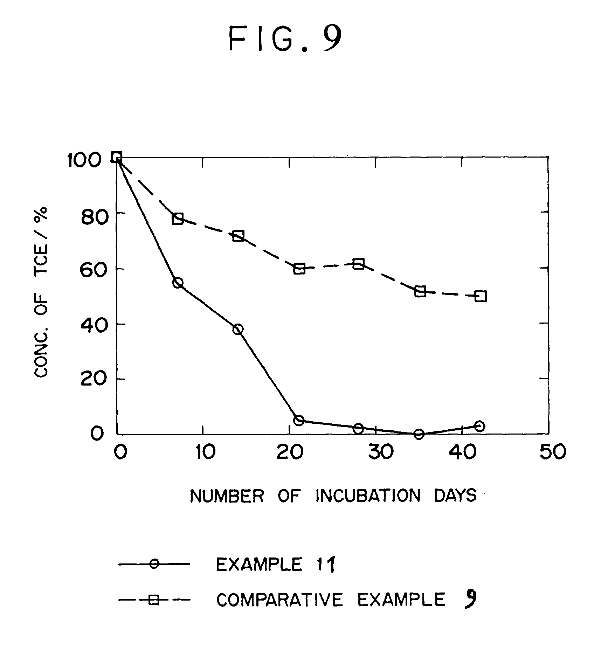

The same procedure as in Example 11 was repeated except that a microorganisms supporting carrier holding a wood powder nutrient was not used, to determine the change of the remaining phenol amount. The obtained results are shown in Fig. 9.

### Example 12

### [Purification of a contaminated soil (3)]

A brown forest soil was mixed with trichloroethylene in such a ratio as to disperse 8 ml of a 60 ppm aqueous trichloroethylene solution to 500 g of the soil, thereby preparing a trichloroethylene-contaminated soil. An underground tank having a length of 2 m, a width of 2 m and a depth of 3 m was filled with this contaminated soil until a layer thickness of 1 m had been reached, and a brown forest soil was superposed upon this contaminated soil layer to form a soil layer (two-layer structure) having a depth of 2.5 m.

Next, water supply holes having a depth of 2.5 m and a diameter of 5 cm were provided at four corners in this test soil tank, and pipes were inserted into these water supply holes. These pipes were made of polyvinyl chloride, had an external diameter of 50 mm, and were provided with many water supply pits having a diameter of 5 mm within the range of 1 m from its tip. The outer periphery of each pipe inlet was sealed with clay to form water supply lines. Furthermore, a pipe having a diameter of 50 mm and many water supply pits within the range of 1 m from its tip was disposed at the central position of the tank in like manner to provide a water suction line. The above-mentioned water supply lines were connected to a water supply pump, and water was supplied at about 0.5 liter/min. In addition, the water suction line is connected to a water suction pump, and water was sucked at about 5 liters/min.

On the other hand, 100 g of a microorganisms supporting carrier holding a cellulose nutrient which was prepared in the same manner as in Example 5 was dispersed in 5 liters of a culture medium (which was prepared by adding 0.05% of yeast extract to an M9 culture medium), and a Pseudomonas cepacia KK01 strain was inoculated into the culture medium, followed by stationary culture for 48 hours. This microorganism culture medium containing the soil remediating agent was inoculated into the prepared soil layer in the soil tank through the water supply lines. This inoculation was given every day over one week.

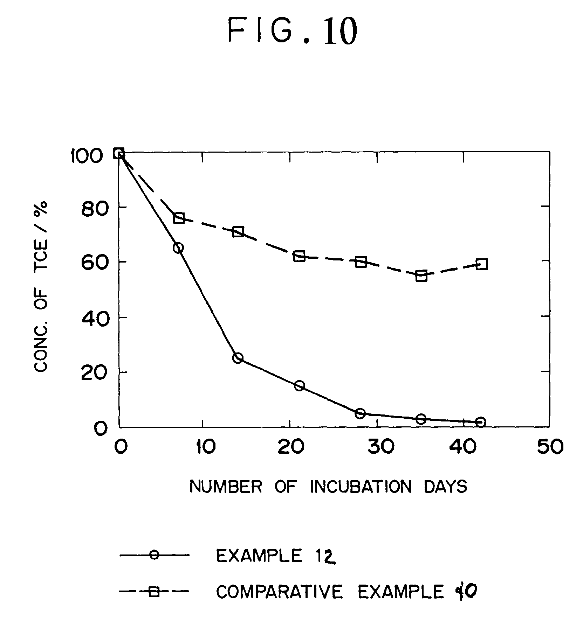

After the inoculation, the soil (at 5 positions in all) was sampled in the vicinity of the water supply lines every one week, and a trichloroethylene concentration in the soil was quantitatively analyzed by a solvent extraction method and a remaining trichloroethylene amount was determined from average values at the 5 positions of the soil. The obtained results are shown in Fig. 10.

### Comparative Example 10

The same procedure as in Example 12 was repeated except that a microorganisms supporting carrier holding a cellulose nutrient was not used, to determine the change of the remaining phenol amount. The obtained results are shown in Fig. 10.

According to the present invention, some effects can be obtained. That is, a microorganisms-supporting carrier for remediating a soil of the present invention can be prepared by an easy and economical process, has many pores suitable to support the microorganisms therein, and can provide a nutrient together. In consequence, the growth power of the microorganisms can be maintained in the soil.

## Claims

1. A soil remedying agent comprising a porous carrier which holds microorganisms capable of degrading a soil contaminant, and a nutrient for the microorganisms,
**characterized in that**
the agent is made by the steps of
(i) incorporating the nutrient into pores of the porous carrier and consolidating the nutrient in the pores; and
(ii) making the microorganisms held on the porous carrier resulted from the step (i).

2. The soil remedying agent according to claim 1, wherein said carrier is biodegradable.

3. The soil remedying agent according to claim 1, wherein the nutrient is a culture medium for the microorganisms.

4. The soil remedying agent according to any one of the preceding claims, wherein at least one of the microorganisms is a microorganism capable of degrading trichloroethylene.

5. The soil remedying agent according to claim 4, wherein the microorganism capable of degrading trichloroethylene is *Pseudomonas cepacia* KK 01.

6. A process of remedying a soil contaminated with a contaminant comprising a step of applying a soil remedying agent comprising a porous carrier which holds microorganisms capable of degrading the contaminant, and a nutrient for the microorganisms, **characterized in that**
the soil remedying agent is provided by the steps of
(i) incorporating the nutrient into pores of the porous carrier and consolidating the nutrient in the pores; and
(ii) making the microorganisms held on the porous carrier resulted from the step (i).

7. The process according to claim 6, wherein the soil remedying agent is applied by providing a water supply hole in the soil, and then feeding a solution containing the soil remedying agent to said soil through the water supply hole to diffuse the soil remedying agent into the soil.

8. The process according to claim 7, wherein the solution containing the soil remedying agent is fed to an underground water vein.

9. The process according to claim 6, wherein the soil remedying agent is applied by providing an aeration hole in the soil, and then feeding a gas containing the soil remedying agent to the soil through the aeration hole to diffuse the soil remedying agent into the soil.

10. A process for remedying a soil comprising the steps of:
(a) providing a soil containing a contaminant;
(b) providing a soil remedying agent by the steps of:
(i) providing a porous carrier;
(ii) immersing the porous carrier into a liquid containing a nutrient for microorganisms capable of degrading the contaminant, and then incorporating the nutrient into pores of the porous carrier;
(iii) drying the porous carrier in which the nutrient has been incorporated into the pores to consolidate the nutrient in the pores;
(iv) immersing the porous carrier in which the nutrient has been consolidated into a liquid containing the microorganisms, and then incorporating the microorganisms into the pores in which the consolidated nutrient has been incorporated to obtain the soil remedying agent; and
(c) applying the soil remedying agent to the soil to degrade the contaminant.

11. The process according to claim 10, wherein the microorganisms comprise a bacteria.

12. The process according to claim 11, wherein the bacteria is capable of degrading trichloroethylene.

13. The process according to claim 12, wherein the bacteria is *Pseudomonas cepacia* KK 01.

## Patentansprüche

1. Mittel zur Bodensanierung, das einen porösen Träger umfaßt, der Mikroorganismen, die zum Abbau eines Bodenschadstoffs geeignet sind, und einen Nährstoff für die Mikroorganismen trägt,
**dadurch gekennzeichnet, daß**
das Mittel durch die nachstehenden Schritte hergestellt wird:
(i) Einarbeiten des Nährstoffs in die Poren des porösen Trägers und Verfestigen des Nährstoffs in den Poren; und
(ii) Veranlassen, daß die Mikroorganismen auf dem aus Schritt (i) resultierenden porösen Träger getragen werden.

2. Mittel zur Bodensanierung nach Anspruch 1,
**dadurch gekennzeichnet, daß**
der Träger biologisch abbaubar ist.

3. Mittel zur Bodensanierung nach Anspruch 1,
**dadurch gekennzeichnet, daß**
der Nährstoff ein Kulturmedium für die Mikroorganismen ist.

4. Mittel zur Bodensanierung nach einem der vorstehenden Ansprüche,
**dadurch gekennzeichnet, daß**
mindestens einer der Mikroorganismen ein Mikroorganismus ist, der zum Abbau von Trichlorethylen geeignet ist.

5. Mittel zur Bodensanierung nach Anspruch 4,
**dadurch gekennzeichnet, daß**
der Mikroorganismus, der zum Abbau von Trichlorethylen geeignet ist, *Pseudomonas cepacia* KK 01 ist.

6. Verfahren zur Sanierung eines Bodens, der mit einem Schadstoff kontaminiert ist, das den Schritt der Verabreichung eines Mittels zur Bodensanierung umfaßt, das einen porösen Träger umfaßt, der Mikroorganismen, die zum Abbau des Schadstoffs geeignet sind, und einen Nährstoff für die Mikroorganismen trägt,
**dadurch gekennzeichnet, daß**
das Mittel zur Bodensanierung durch die nachstehenden Schritte zur Verfügung gestellt wird:
(i) Einarbeiten des Nährstoffs in die Poren des porösen Trägers und Verfestigen des Nährstoffs in den Poren; und
(ii) Veranlassen, daß die Mikroorganismen auf dem aus Schritt (i) resultierenden porösen Träger getragen werden.

7. Verfahren nach Anspruch 6,
**dadurch gekennzeichnet, daß**
das Mittel zur Bodensanierung dadurch verabreicht wird, daß der Boden mit einem Wasserzufuhrloch versehen wird, und eine Lösung, die das Mittel zur Bodensanierung enthält, dem Boden anschließend durch das Wasserzufuhrloch zugeführt wird, um das Mittel zur Bodensanierung in dem Boden zu verteilen.

8. Verfahren nach Anspruch 7,
**dadurch gekennzeichnet, daß**
die Lösung, die das Mittel zur Bodensanierung enthält, einer unterirdischen Wasserader zugeführt wird.

9. Verfahren nach Anspruch 6,
**dadurch gekennzeichnet, daß**
das Mittel zur Bodensanierung dadurch verabreicht wird, daß der Boden mit einem Belüftungsloch versehen wird, und ein Gas, das das Mittel zur Bodensanierung enthält, dem Boden anschließend durch das Belüftungsloch zugeführt wird, um das Mittel zur Bodensanierung in dem Boden zu verteilen.

10. Verfahren zur Bodensanierung, das die nachstehenden Schritte umfaßt:
(a) Bereitstellung eines Bodens, der einen Schadstoff enthält;
(b) Bereitstellung eines Mittels zur Bodensanierung durch die nachstehenden Schritte:
(i) Bereitstellung eines porösen Trägers;
(ii) Eintauchen des porösen Trägers in eine Flüssigkeit, die einen Nährstoff für Mikroorganismen, die zum Abbau des Schadstoffs geeignet sind, enthält, und eine anschließende Einarbeitung des Nährstoffs in die Poren des porösen Trägers;
(iii) Trocknen des porösen Trägers, in dem der Nährstoff in die Poren eingearbeitet wurde, um den Nährstoff in den Poren zu verfestigen;
(iv) Eintauchen des porösen Trägers, in dem der Nährstoff verfestigt wurde, in eine Flüssigkeit, die die Mikroorganismen enthält, und eine anschließendes Einarbeitung der Mikroorganismen in die Poren, in die der verfestigte Nährstoff eingearbeitet wurde, um das Mittel zur Bodensanierung zu erhalten; und
(c) Verabreichung des Mittels zur Bodensanierung an den Boden, um den Schadstoff abzubauen.

11. Verfahren nach Anspruch 10,
**dadurch gekennzeichnet, daß**
die Mikroorganismen ein Bakterium umfassen.

12. Verfahren nach Anspruch 11,
**dadurch gekennzeichnet, daß**
das Bakterium zum Abbau von Trichlorethylen geeignet ist.

13. Verfahren nach Anspruch 12,
**dadurch gekennzeichnet, daß**
es sich bei dem Bakterium um *Pseudomonas cepacia* KK 01 handelt.

## Revendications

1. Agent de régénération du sol, comprenant un support poreux qui renferme des micro-organismes capables de dégrader un contaminant présent dans le sol, et une substance nutritive pour les micro-organismes,
**caractérisé en ce que**
l'agent est préparé par les étapes consistant
(i) à incorporer la substance nutritive aux pores du support poreux et à consolider la substance nutritive dans les pores ; et
(ii) à incorporer les micro-organismes au support poreux résultant de l'étape (i).

2. Agent de régénération du sol suivant la revendication 1, dans lequel le support est biodégrable.

3. Agent de régénération du sol suivant la revendication 1, dans lequel la substance nutritive est un milieu de culture pour les micro-organismes.

4. Agent de régénération du sol suivant l'une quelconque des revendications précédentes, dans lequel au moins un des micro-organismes est un micro-organisme capable de dégrader le trichloréthylène.

5. Agent de régénération du sol suivant la revendication 4, dans lequel le micro-organisme capable de dégrader le trichloréthylène est *Pseudomonas cepacia* KK 01.

6. Procédé pour régénérer un sol contaminé par un contaminant, comprenant une étape consistant à appliquer un agent de régénération du sol comprenant un support poreux qui renferme des micro-organismes capables de dégrader le contaminant et une substance nutritive pour les micro-organismes, **caractérisé en ce que**
l'agent de régénération du sol est produit par les étapes consistant
(i) à incorporer la substance nutritive aux pores du support poreux et à consolider la substance nutritive dans les pores ; et
(ii) à incorporer les micro-organismes au support poreux résultant de l'étape (i).

7. Procédé suivant la revendication 6, dans lequel l'agent de régénération du sol est appliqué en creusant dans le sol un trou de distribution d'eau, puis en introduisant une solution contenant l'agent de régénération du sol dans ce sol par le trou de distribution d'eau pour faire diffuser l'agent de régénération du sol dans le sol.

8. Procédé suivant la revendication 7, dans lequel la solution contenant l'agent de régénération du sol est introduite dans un courant d'eau souterrain.

9. Procédé suivant la revendication 6, dans lequel l'agent de régénération du sol est appliqué en creusant un trou d'aération dans le sol, puis en introduisant un gaz contenant l'agent de régénération du sol dans le sol par le trou d'aération pour faire diffuser l'agent de régénération du sol dans le sol.

10. Procédé pour régénérer un sol, comprenant les étapes consistant :
(a) à choisir un sol contenant un contaminant ;
(b) à préparer un agent de régénération du sol par les étapes consistant :
(i) à prendre un support poreux ;
(ii) à immerger le support poreux dans un liquide contenant une substance nutritive pour des micro-organismes capables de dégrader le contaminant, puis incorporer la substance nutritive aux pores du support poreux ;
(iii) à sécher le support poreux dans lequel la substance nutritive a été incorporée dans les pores pour consolider la substance nutritive dans les pores ;
(iv) à immerger le support poreux dans lequel la substance nutritive a été consolidée dans un liquide contenant les micro-organismes, et ensuite incorporer les micro-organismes aux pores dans lesquels la substance nutritive consolidée a été incorporée pour obtenir l'agent de régénération du sol ; et
(c) à appliquer l'agent de régénération du sol au sol pour dégrader le contaminant.

11. Procédé suivant la revendication 10, dans lequel les micro-organismes comprennent une bactérie.

12. Procédé suivant la revendication 11, dans lequel la bactérie est capable de dégrader le trichloréthylène.

13. Procédé suivant la revendication 12, dans lequel la bactérie est *Pseudomonas cepacia* KK 01.
